# EUROPEAN PATENT APPLICATION

(11) **EP 4 006 553 A1**
(43) Date of publication of application: **01.06.2022**
(21) Application number: 20844790.4
(22) Date of filing: 08.07.2020
(51) Int. Cl.: G01N 33/80, G01N 33/577

(54) **BLOOD GROUP ANTIGEN TESTING COMPONENT**

(30) Priority: 23.07.2019 CN 201921168987 U
(71) Applicant: Intec Products, Inc., Xiamen, Fujian 361000 (CN)
(72) Inventor: HU, Jinggao, Xiamen, Fujian 361000 (CN)
(74) Representative: Verscht, Thomas Kurt Albert
(86) International application number: PCT/CN2020/100784
(87) International publication number: WO 2021/012948

(57) **Abstract**

Disclosed is a blood group antigen testing component comprising a test strip (1) and a device for sample loading (2), wherein a sample is transported to a test area (16) of the test strip (1) through a sampling ring (22) of the device for sample loading (2). A known blood group antibody is used for identifying a human blood group antigen by means of an immunochromatography technique. Firstly, the antibody is pre-coated and immobilized on a reaction membrane (11) to present a test point, and then the test point, a flushing pad (12), absorbent paper and a liner are assembled into a test strip; when a sample is tested, the sample is added dropwise into the reaction membrane (11), then a sample flushing fluid is added dropwise into the flushing pad (12), and when red blood cells in the sample to be tested and the immobilized antibody are subjected to an immunobinding reaction, the red blood cells are intercepted at a test point on the reaction membrane (11), showing a red point, for a positive reaction; and if there is no antigen-antibody immune reaction, the red blood cells cannot be intercepted, but move forwards to enter an absorbent pad (13) by means of the effect of chromatography, the test point on the reaction membrane (11) shows white, with only the positive control point showing red, for a negative reaction.

## Description

### FIELD OF THE DISCLOSURE

The present disclosure relates to a detection component for blood group antigens.

### BACKGROUND OF THE DISCLOSURE

Blood groups of red blood cells are classified according to surface antigens of the red blood cells in the blood. According to various types of blood group antigens, ISBT reported that there are 29 groups and 221 antigens in 2004, and each blood group is independently inherited. The ABO blood group and the RhD blood group are two of the most important blood groups in humans and have important clinical implications due to high immunogenicity, especially closely related to clinical blood transfusion, organ transplantation, etc. Correct blood group testing is important work to ensure safety of the clinical blood transfusion and rescue of life. At present, tests of the ABO blood group and the RhD blood group are routine blood group serological test items necessary before blood transfusion.

In the existing techniques, the commonly used clinical testing methods for blood group antigens comprise an agglutination method, a microcolumn gel method, and a solid-phase card method. Reagents used in the agglutination method need refrigerated transport at 2-8 °C, the results cannot be standardized without control reagents, and a centrifuge or a glass slide is further required for the reaction. The operation is cumbersome, the results are greatly affected by subjective deviations, easily leading to errors in the blood group testing, and there might be problems, by way of example, contamination possibilities of liquid reagents and blood samples. In the microcolumn gel method, special gel cards are required. The costs of gel and blank card are relatively high. Further, a sample to be tested needs to be pre-processed to be configured into an about 1% red blood cell suspension. At the same time, a special centrifuge is required, and the reaction time is long, usually 5-10 minutes. Therefore, it has disadvantages of complicated operation, time-consuming, labor-intensive, cost-consuming, etc. Blood group antigen testing using the solid-phase card method is a newly emerged product in recent years. The main problem is that there is no control; the results cannot be standardized and are greatly affected by subjective deviations, the card is large in size, a lot of medical wastes are easily created, and the cost is high. When the blood group antigens are tested, each test strip is relatively independent. When various blood group antigens are tested, it is necessary to separately add samples to a sample addition section of each test strip and to separately add flushing liquid to a flushing section of each test strip. Users need to drip samples or flush liquids in multiple sections. The operation is complicated and is prone to errors. At the same time, the sample volume is large.

In sum, the testing methods for the blood group in the existing techniques have problems comprising high manual labor intensity, long testing time, high cost, the results are more affected by subjective deviations, the possibility of contamination, etc. Therefore, it is necessary to make substantial improvements to traditional testing methods for blood group.

### BRIEF SUMMARY OF THE DISCLOSURE

An objective of the present disclosure is to provide a detection component for blood group antigens to overcome deficiencies of existing techniques.

A technical solution of the present disclosure is as follows.

A detection component for blood group antigens comprises:
A test strip comprising a base board, wherein a reaction membrane, a flushing pad, a water absorption pad and a sealing pad are disposed on the base board, the reaction membrane is disposed on a middle portion of a loader, one end of the flushing pad overlaps another end of the reaction membrane, another end of the water absorption pad overlaps one end of the reaction membrane, one part of the sealing pad is stuck to the reaction membrane, another part is stuck to the flushing pad, a section of the reaction membrane disposed between one end of the sealing pad and the another end of the water absorption pad defines a testing section, an A testing point, a B testing point, a D testing point and a Ctl testing point are disposed on the testing section, the A testing point is coated with a solidified anti-A monoclonal antibody, the B testing point is coated with a solidified anti-B monoclonal antibody, the D testing point is coated with a solidified anti-D monoclonal antibody, the Ctl testing point is coated with a solidified anti-RBC monoclonal antibody;
A sample addition device comprising a handle and a sampling ring, wherein the sampling ring is disposed on one end of the handle, a length of the sampling ring is less than a width of the reaction membrane while is sufficient to cover the A testing point, the B testing point, the D testing point and the Ctl testing point;
An inner side wall of the sampling ring comprises a plurality of protrusions equally spaced along a circumference of the inner side wall of the sampling ring to increase a surface tension force of the sampling ring and to ensure a sampling volume of 10-20 µL; and
A sample is transported to the testing section of the test strip by the sampling ring of the sample addition device.

In a preferred embodiment of the present disclosure, a tag is disposed on the water absorption pad.

In a preferred embodiment of the present disclosure, the A testing point, the B testing point, the D testing point and the Ctl testing point are linearly arrayed or staggered up and down to be shaped as an "N" along a width direction of the testing section.

In a preferred embodiment of the present disclosure, the sampling ring is shaped as an ellipse or a rectangle.

In a preferred embodiment of the present disclosure, the sampling ring is shaped as a rectangle having a circular angle.

In a preferred embodiment of the present disclosure, the reaction membrane is a nitrocellulose membrane, a cellulose acetate membrane, a polyethylene membrane or a nylon membrane.

In a preferred embodiment of the present disclosure, the reaction membrane is a nitrocellulose membrane having a back support, having a capillary flow rate of less than 90 seconds, and a width of 4 cm.

The present disclosure has the following advantages.
1. The present disclosure uses immunochromatography technology to identify human blood group antigens with existing blood group antibodies. The antibodies are pre-coated and solidified on the reaction membrane to define testing points and then assembled to the flushing pad, a water absorption paper and a pad to define the test strip. When a sample is tested (anticoagulated whole blood, blood from a fingertip, a suspension of red blood cells), the sample is dripped on the reaction membrane, and the flushing solution for the sample is then dripped on the flushing pad. When the red blood cells in the sample to be tested are reacted with the antibodies of a solid-phase due to immunoconjugate, the red blood cells are retained at the testing points disposed on the reaction membrane and define a red dot, which indicates a positive reaction and indicates that surfaces of the red blood cells have the correlated blood group antigens. When antigen-antibody immune response does not occur, the red blood cells cannot be retained and move forward into the water absorption pad due to chromatography function, the testing points disposed on the reaction membrane are white in color, and only the positive control point is red in color, which indicates a negative reaction.
2. The design is compact, the integration is high, the volume is small, and the product cost is low, a test for the ABO blood group is completed using one drop of sample, manual operation is simplified during testing, false testing caused by manual errors is reduced, and medical waste is less due to no card sleeve design.
3. In the present disclosure, the transportation and the storage are convenient. It is stored at room temperature (2-30°C), the validity period is long (24 months), the sensitivity is high, it is a solid-phase test strip and is portable, the testing time is shortened to 1 minute, the operation is simple, positive control is provided, interpretation of results is easy, objective and reliable, and no auxiliary equipment is required.

### BRIEF DESCRIPTION OF THE DRAWINGS

Fig. 1 illustrates a structural view of a test strip of the present disclosure.
Fig. 2 illustrates a structural view of a sample addition device of the present disclosure.

### DETAILED DESCRIPTION OF THE EMBODIMENTS

The present disclosure will be further described in combination with the accompanying embodiments and drawings.

A detection component for blood group antigens comprises a test strip 1 and a sample addition device 2.

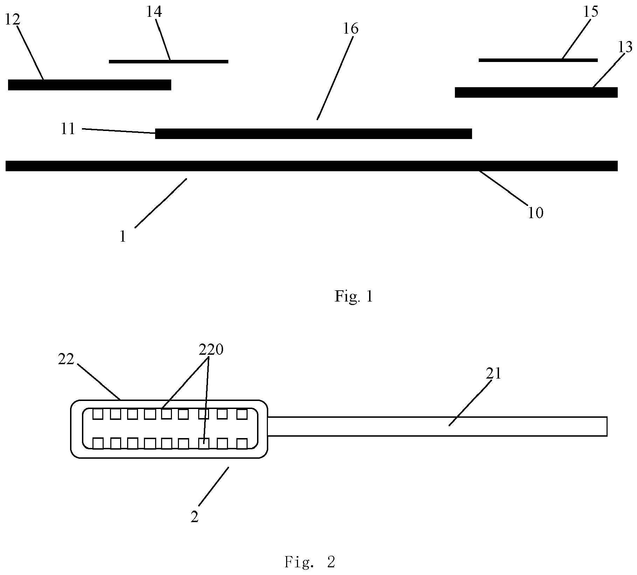

Referring to Fig. 1, the test strip 1 comprises a base board 10, and a reaction membrane 11, a flushing pad 12, a water absorption pad 13 and a sealing pad 14 are disposed on the base board 10. The reaction membrane 11 is disposed on a middle portion of a loader, and one end of the flushing pad 12 overlaps another end of the reaction membrane 11. Another end of the water absorption pad 13 overlaps one end of the reaction membrane 11, and a tag 15 is stuck to an upper side of the water absorption pad 13. One part of the sealing pad 14 is stuck to the reaction membrane 11, and another part is stuck to the flushing pad 12. A section of the reaction membrane 11 disposed between one end of the sealing pad 14 and the another end of the water absorption pad 13 defines a testing section 16. The testing section 16 integrates an observation window and a sample addition section on which an A testing point, a B testing point, a D testing point, and a Ctl testing point are disposed. The A testing point is coated with a solidified anti-A monoclonal antibody and defines a light cyan color. The B testing point is coated with a solidified anti-B monoclonal antibody and defines a light yellow color. The D testing point is coated with a solidified anti-D monoclonal antibody and defines a light red color. The Ctl testing point is coated with a solidified anti-RBC monoclonal antibody and defines a light blue color. The A testing point, the B testing point, the D testing point, and the Ctl testing point are linearly arrayed or staggered up and down to be shaped as an "N" along a width direction of the testing section 16.

The reaction membrane 11 is a nitrocellulose membrane, a cellulose acetate membrane, a polyethylene membrane, or a nylon membrane. Preferably, a nitrocellulose membrane having a back support, having a capillary flow rate of less than 90 seconds, and a width of 4 cm, and a pore size of the nitrocellulose membrane is preferably selected such that a single red blood cell can penetrate into the porous structure and move chromatographically.

The flushing pad 12 is made of porous polymer material having water conductivity and hydrophilicity, preferably, glass fiber.

The water absorption pad 13 is made of absorbent materials, preferably, cotton pulp paper.

Referring to Fig. 2, the sample addition device 2 comprises a handle 21 and a sampling ring 22. The sampling ring 22 is disposed on one end of the handle 21. A length of the sampling ring 22 is less than a width of the reaction membrane 11 while is sufficient to cover the A testing point, the B testing point, the D testing point and the Ctl testing point. An inner side wall of the sampling ring 22 comprises a plurality of protrusions 220 equally spaced along a circumference of the inner side wall of the sampling ring 22 to increase a surface tension force of the sampling ring 22 and to ensure a sampling volume of 10-20 µL. The handle 21 is solid, and a cross-section can be circular and polygonal, preferably, circular.

In a preferred embodiment, a shape of the sampling ring 22 defines a rectangle shape having circular angles.

A sample is transported to the testing section 16 of the test strip 1 by the sampling ring 22 of the sample addition device 2.

A method of the present disclosure is applied as follows: the sample to be tested (anticoagulated whole blood, blood from a fingertip, a suspension of red blood cells) is added to the testing section 16 by the sampling ring 22, a flushing solution is added to the flushing pad 12, and results are observed after about 1 minute. When the red blood cells in the sample to be tested are reacted with the antibodies of the solid-phase by immunoconjugate, the red blood cells are retained on the testing points on the reaction membrane 11 (i.e., points pre-coated with antibodies) and define a red dot, which indicates a positive reaction and indicates that surfaces of the red blood cells comprise the correlated blood group antigens. When antigen-antibody immune response does not occur, the red blood cells cannot be retained and move forward into the water absorption pad 13 due to chromatography function, the testing points on the reaction membrane 11 are white in color, and only the positive control point is red in color, which indicates a negative reaction.

The aforementioned embodiments are merely some embodiments of the present disclosure, and the scope of the disclosure is not limited thereto. Thus, it is intended that the present disclosure cover any modifications and variations of the presently presented embodiments provided they are made without departing from the appended claims and the specification of the present disclosure. By way of example: 1. the anti-A monoclonal antibody, the anti-B monoclonal antibody, and anti-D monoclonal antibody are replaced with antibodies of other blood groups; and/or 2. testing points for antibodies of other blood groups are added.

### Industrial applicability

The present disclosure provides a detection component for blood group antigens, which comprises a test strip and a sample addition device. A sample is transported to the testing section of the test strip by the sampling ring of the sample addition device. The present disclosure uses immunochromatography technology to identify human blood group antigens with existing blood group antibodies. The antibodies are pre-coated and solidified on the reaction membrane to define testing points and then assembled to the flushing pad, the water absorption paper, and the pad to define the test strip. When a sample is tested, the sample is dripped on the reaction membrane, and the flushing solution for the sample is then dripped on the flushing pad. When the red blood cells in the sample to be tested are reacted with the antibodies of the solid phase due to immunoconjugate, the red blood cells are retained at the testing points disposed on the reaction membrane and define a red dot, which indicates a positive reaction. When antigen-antibody immune response does not occur, the red blood cells cannot be retained and move forward into the water absorption pad due to chromatography function, the testing points disposed on the reaction membrane are white in color, and only the positive control point is red in color, which indicates a negative reaction. The industrial applicability is good.

## Claims

1. A detection component for blood group antigens, **characterized in that**, which comprises:
a test strip comprising a base board, wherein a reaction membrane, a flushing pad, a water absorption pad and a sealing pad are disposed on the base board, the reaction membrane is disposed on a middle portion of the base board, one end of the flushing pad overlaps another end of the reaction membrane, another end of the water absorption pad overlaps one end of the reaction membrane, one part of the sealing pad is stuck to the reaction membrane, another part is stuck to the flushing pad, a section of the reaction membrane disposed between one end of the sealing pad and the another end of the water absorption pad defines a testing section, an A testing point, a B testing point, a D testing point, and a Ctl testing point are disposed on the testing section, the A testing point is coated with a solidified anti-A monoclonal antibody, the B testing point is coated with a solidified anti-B monoclonal antibody, the D testing point is coated with a solidified anti-D monoclonal antibody, and the Ctl testing point is coated with a solidified anti-RBC monoclonal antibody; and
a sample addition device, which comprises a handle and a sampling ring, wherein the sampling ring is disposed on one end of the handle, a length of the sampling ring is less than a width of the reaction membrane while is sufficient to cover the A testing point, the B testing point, the D testing point, and the Ctl testing point, an inner side wall of the sampling ring comprises a plurality of protrusions equally spaced along a circumference of the inner side wall of the sampling ring to increase a surface tension force of the sampling ring and to ensure a sampling volume of 10-20 µL; and
a sample is transported to the testing section of the test strip by the sampling ring of the sample addition device.

2. The detection component for the blood group antigens according to claim 1, **characterized in that**: a tag is disposed on the water absorption pad.

3. The detection component for the blood group antigens according to claim 1, **characterized in that**: the A testing point, the B testing point, the D testing point and the Ctl testing point are linearly arrayed or staggered up and down to be shaped as an "N" along a width direction of the testing section.

4. The detection component for the blood group antigens according to claim 1, **characterized in that**: the sampling ring is shaped as an ellipse or a rectangle.

5. The detection component for the blood group antigens according to claim 4, **characterized in that**: the sampling ring is shaped as a rectangle having a circular angle.

6. The detection component for the blood group antigens according to claim 1, **characterized in that**: the reaction membrane is a nitrocellulose membrane, a cellulose acetate membrane, a polyethylene membrane or a nylon membrane.

7. The detection component for the blood group antigens according to claim 6, **characterized in that**: the reaction membrane is a nitrocellulose membrane having a back support, having a capillary flow rate of less than 90 seconds, and a width of 4 cm.

8. A detection kit for blood group antigens, **characterized in that**: which comprises:
a test strip comprising a base board, wherein a reaction membrane, a flushing pad, a water absorption pad and a sealing pad are disposed on the base board, the reaction membrane is disposed on a middle portion of the base board, the flushing pad is disposed on one side of the reaction membrane with one end overlapping the reaction membrane, the water absorption pad is disposed on another side of the reaction membrane, one end of the water absorption pad overlaps another end of the reaction membrane, one part of the sealing pad is stuck to the reaction membrane, another part is stuck to the flushing pad, a section of the reaction membrane disposed between one end of the sealing pad and another end of the water absorption pad defines a testing section, an A testing point, a B testing point, a D testing point and a Ctl testing point are disposed on the testing section, the A testing point is coated with a solidified anti-A monoclonal antibody, the B testing point is coated with a solidified anti-B monoclonal antibody, the D testing point is coated with a solidified anti-D monoclonal antibody, and the Ctl testing point is coated with a solidified anti-RBC monoclonal antibody; and
a sample addition device comprising a sampling ring, wherein the sampling ring is configure to add a sample to the A testing point, the B testing point, the D testing point and the Ctl testing point at a same time.

9. The detection kit for the blood group antigens according to claim, **characterized in that**: the sampling ring is disposed with a handle and a plurality of protrusions equally spaced and configured to correspond to testing points on the reaction membrane.

10. The detection component for the blood group antigens according to claim 8 or 9, **characterized in that**: a tag is disposed on the water absorption pad.

11. The detection component for the blood group antigens according to claim 8 or 9, **characterized in that**: the A testing point, the B testing point, the D testing point and the Ctl testing point are linearly arrayed or staggered up and down to be shaped as an "N" along a width direction of the testing section.
